(19) Europäisches Patentamt
European Patent Office
Office européen des brevets

(11) Publication number: 0 351 244 A2

(12) **EUROPEAN PATENT APPLICATION**

(21) Application number: 89307188.6

(22) Date of filing: 14.07.89

(51) Int. Cl.5: C 12 P 21/08
G 01 N 33/543, G 01 N 33/68

(30) Priority: 15.07.88 JP 177878/88

(43) Date of publication of application:
17.01.90 Bulletin 90/03

(84) Designated Contracting States:
AT BE CH DE ES FR GB GR IT LI LU NL SE

(71) Applicant: TAKEDA CHEMICAL INDUSTRIES, LTD.
3-6, Doshomachi 2-chome Chuo-ku
Osaka 541 (JP)

(72) Inventor: Kondo, Koichi
6-3, Kabutodai 3-chome Kizu-cho
Soraku-gun Kyoto 619-02 (JP)

Wakimasu, Mitsuhiro
502, 17-1, Namiki 3-chome
Tsukuba Ibaraki 305 (JP)

Watanabe, Hiroyuki
107, 1-6, Minamikoshien 3-chome
Nishinomiya Hyogo 663 (JP)

(74) Representative: Lewin, John Harvey et al
ELKINGTON AND FIFE Beacon House 113 Kingsway
London WC2B 6PP (GB)

(54) **Antibodies and methods for immunochemical assay using them.**

(57) An antibody that recognizes the amino acid sequence between Asp and another amino acid residue in the C-terminal side in the cyclic region of α-ANP. Immunochemical assay of ANPs using this antibody is also disclosed.

EP 0 351 244 A2

## Description

# ANTIBODIES AND METHODS FOR IMMUNOCHEMICAL ASSAY USING THEM

## FIELD OF THE INVENTION

The present invention relates to antibodies and a method for immunochemical assay of ANPs (atrial natriuretic polypeptides) using them.

## BACKGROUND OF THE INVENTION

Recently, three kinds of peptides possessing potent natriuretic activities have been isolated from the human atrium and their structures have been determined. Among these three peptides, the peptide having the lowest molecular weight is composed of 28 amino acid residues and has been named α-human atrial natriuretic polypeptide (hereinafter abbreviated as α-hANP) [Biochemical and Biophysical Research Communications, 118, 131-139 (1984)].

The chemical structure of α-hANP is as follows:

```
1               5                   10
H-Ser-Leu-Arg-Arg-Ser-Ser-Cys-Phe-Gly-Gly-Arg-Met-

        15                      20
Asp-Arg-Ile-Gly-Ala-Gln-Ser-Gly-Leu-Gly-Cys-Asn-

25
Ser-Phe-Arg-Tyr-OH
```

Then, based on the investigation of the structure-activity correlation of α-hANP [7-28], it has been found that an N-terminal shortened derivative of α-hANP has the same natriuretic activities as that of α-hANP.

At almost the same time, another peptide possessing natriuretic activities was isolated from the rat atrium, and its structure was determined. It has been found that this peptide, called α-rANP or Cardionatrin I, has the same amino acid sequence as that of α-hANP except that only the amino acid at 12-position is converted from methionine to isoleucine. Also, there have been found some analogues of α-rANP which lack several amino acid residues at the N-terminal. Thus, among atrial natriuretic peptides (ANPs), α-ANP can be roughly divided into the human type and the rat type.

Since ANP is found in atrial cells and possesses natriuretic activities, hypotensive activities, blood vessel smooth muscle relaxation activities, renin hyposecretion activities and other activities, ANP is now drawing attention as a factor involved in various diseases. That is, it is said that the blood plasma α-hANP level in human beings increases in various heart diseases (e.g. paroxysmal atrial arrhythmia, congestive heart failure), renal failure, liver cirrhosis, primary aldosteronism and some cases of essential hypertension. Therefore, α-hANP has potential for using as a useful marker for diagnosis and prognosis of these diseases.

As methods for determination of ANPs, particularly, α-hANP, in addition to bioassay methods, immunochemical assay methods have been proposed and radioimmunoassay (RIA) and enzyme immunoassay (EIA) have been reported. Some of them have been already employed in practice.

In general, the above methods of immunochemical assay can be roughly divided into the following two types:

(1) Competitive technique: A solution containing an unknown amount of an antigen to be tested and a given amount of a labeled antigen are competitively reacted with a given amount of the corresponding antibody. The activity of the antibody-coupled portion or uncoupled portion of the labeling agent is then measured.

(2) Sandwich technique: A solution containing an unknown amount of an antigen to be tested is reacted with an excess amount of carrier-coupled antibody (first reaction), followed by reaction with a given excess amount of another antibody labeled with a labeling agent (second reaction). The activity of the carrier-captured portion or uncaptured portion of the labeling agent is then measured. The first and second reactions may be carried out simultaneously or at an interval.

For α-hANP, the competitive technique (1) is employed in most cases. However, since α-hANP level in blood in normal human beings is extremely low, i.e., at most dozens of pg/mℓ, α-hANP should be concentrated from a large amount of blood plasma by solvent extraction or column concentration before determination. Further, attempts have recently been made to determine α-hANP by the sandwich technique (2) and it has been reported that α-hANP can be specifically determined by this technique (12th Scientific

Meeting of the International Society of Hypertension, May 22-26, 1988, Kyoto, Japan, Abstracts, Main Meeting 0468; Japanese Patent Laid Open Publication No. 61500/1989).

However, the monoclonal antibody used in the above method based on the sandwich technique does not permit determination of α-rANP, although α-hANP can be determined.

The present inventors have conducted investigations on ANP determination by immunochemical assay, and developed a system that permits determination of both α-hANP and α-rANP. That is, the capability to determine α-hANP and α-rANP with almost equal sensitivity makes it feasible to determination of both human type and rat type α-ANPs using a single kit of assay reagents, which in turn leads to efficient use of a single kit for diagnosis, prognosis and clinical trials in human beings as well as for various animal experiments.

EP-A-0 195 331 discloses monoclonal antibodies for determination of both α-h and -rANPs by the competitive radioimmunoassay [see also Life Sciences, Vol. 38, pp. 1991-1997 (1986); and Klin Wochenschr. (1987) 65:533-537]. However, these antibodies are completely different from that used in the present invention.

EP-A-0 269 220 which has been assigned to the present assignee discloses a process for production of a peptide derivative which is useful for the production of an immunogen in the present invention.

## OBJECTS OF THE INVENTION

One object of the present invention is to provide an antibody useful for immunochemical assay of both α-h and -rANPs by the sandwich technique.

Another object of the present invention is to proved a method of immunochemical assay of ANPs useful for the sandwich technique.

These objects as well as other objects and advantages of the present invention will become apparent to those skilled in the art from the following description with reference to the accompanying drawings.

## BRIEF EXPLANATION OF THE DRAWINGS

Fig. 1 is a graph illustrating the ratio of inhibition of the reaction between α-hANP and the polyclonal antibody (R3) by each competing agent determined in Example 4 hereinafter.

Fig. 2 is a graph illustrating the ratio of inhibition of the reaction between α-hANP and the monoclonal antibody (HA53) by each competing agent determined in Example 5 hereinafter.

Figs. 3 and 4 are graphs illustrating the cross reactivities of α-hANP [13-28], α-hANP [18-28] and CM-α-hANP[1-28] determined in Example 5 hereinafter.

Fig. 5 is a graph illustrating the relation between the absorbance at 492 nm versus α-hANP and α-rANP concentrations determined by the assay method of Example 6 hereinafter.

## SUMMARY OF THE INVENTION

According to the present invention, there are provided:

(1) An antibody which recognizes the amino acid sequence between Asp and another amino acid residue in the C-terminal side in the cyclic region of α-ANP;

(2) A method of immunochemical assay of ANPs which comprises using both the antibody of the above (1) and another antibody whose antigen determinant site differs from that of the former; and

(3) An antibody obtained by using as the immunogen a coupled product of a peptide derivative represented by the general formula (I):

R-Cys-Phe-Gly-Gly-Arg-$R^1$-Asp-Arg-Ile-Gly-Ala-Gln-

Ser-Gly-Leu-Gly-Cys-Asn-Ser-Phe-Arg-Tyr-OH

(I)

wherein R is hydrogen, Ser, Ser-Ser, Arg-Ser-Ser, Arg-Arg-Ser-Ser, Leu-Arg-Arg-Ser-Ser, Ser-Leu-Arg-Arg-Ser-Ser or hydrocarbon acyl having 2 to 18 carbon atoms which may be substituted with an amino group at the α-position; $R^1$ represents Met or Ile, and a high molecular carrier material.

## DETAILED DESCRIPTION OF THE INVENTION

The abbreviations of amino acids and peptides used herein are those conventionally used in this art or those employed by the IUPAC-IUB Commission on Biochemical Nomenclature including the abbreviations listed below. The optical configuration of each amino acid is the L-configuration unless otherwise stated.

Ala: alanine
Asp: aspartic acid
Asn: asparagine
Cys: cysteine
Gln: glutamine
Phe: phenylalanine
Gly: glycine
Ile: isoleucine
Leu: leucine
Met: methionine
Ser: serine
Tyr: tyrosine
Arg: arginine
Lys: lysine
Val: valine
Aib: α-aminoisobutyric acid

The above-described α-ANPs include α-hANP, α-rANP, the peptides lacking 1 to 6 amino acid residues at N-terminal of these peptide hormones and the peptide derivatives represented by the above formula (I).

The hydrocarbon acyl having 2 to 18 carbon atoms which may be substituted with an amino group at α-position, for R in the formula (I) may be aliphatic, aromatic or aliphatoaromatic one. It is preferred that the hydrocarbon acyl has 3 to 5 carbon atoms.

The aliphatic hydrocarbon acyl may be saturated or unsaturated one and may be straight, branched or cyclic one. Examples of aliphatic hydrocarbon acyl include acetyl, propionyl, butyryl, isobutyryl, valeryl, isovaleryl, pivaloyl, caproyl, caprylyl, capryl, lauroyl, myristoyl, palmitoyl, stearoyl, cyclobutanecarbonyl, cyclopentanecarbonyl, cyclohexanecarbonyl, oleoyl and linoleyl.

Examples of the aromatic hydrocarbon acyl or aliphatoaromatic hydrocarbon acyl include benzoyl, phenylacetyl and phenylpropionyl.

Examples of the hydrocarbon acyl substituted by an amino group at the α-position include amino acid residues such as Ala, Val, Leu, Ile, nor-Leu, Phe, Lys, Aib, 1-amino-1-cyclobutanecarboxylic acid, 1-amino-1-cyclopentanecarboxylic acid, 1-amino-1-cyclohexanecarboxylic acid, 2- phenyl-Gly, 2-aminocaproic acid and 2-aminocapric acid. When an optical isomer exists in the amino acid, it may be either L- or D-isomer.

These peptides or peptide derivatives can be produced by a conventional method in the field of peptide synthesis regardless of the solid phase method or liquid phase method. Examples of the method of peptide synthesis include the methods described by Schroder and Lubke, "The Peptides", Vol. 1 (1966), Academic Press, New York, U.S.A.; Izumiya et al., "Peptide Gosei (Peptide Synthesis)", Maruzen (1975); Izumiya et al., "Peptide Gosei no Kiso to Jikken (Fundamentals and Experiments of Peptide Synthesis)", Maruzen (1985) and EP-A-0 269 220.

Examples of the above-described ANP include a peptide or peptide derivative containing the fragment between the 7th amino acid residue, Cys, and the 28th amino acid residue, Tyr, in α-hANP or α-rANP, in which two Cys residues in said fragment are bound together to form a cyclic structure. Specific examples of such ANPs are α-hANP, β-hANP, γ-hANP, α-rANP, β-rANP, γ-rANP and peptide derivatives represented by the above formula (I). Among them, α-hANP and α-rANP are preferred.

In the method of immunochemical assay of these ANPs, it is preferred to use the sandwich technique, although it is also possible to carry out the assay based on the competitive technique.

Hereinafter, the antibodies of the present invention are illustrated.

The antibody which recognizes the amino acid sequence between Asp and another amino acid residue in the C-terminal side in the cyclic region of α-ANP of the present invention (hereinafter sometimes referred to as antibody A) recognizes all or a part of the amino acid sequence between the 13th amino acid residue, Asp, and the 23th amino acid residue, Cys, in α-ANP, preferably, a fragment composed of, at least, 5 amino acid residues in the amino acid sequence.

The antibody whose antigen determinant site differs from that of the antibody A used for the method of immunochemical assay of ANPs of the present invention is preferably an antibody that recognizes the sequence of about ten amino acids in the C-terminal region of α-ANP (hereinafter sometimes referred to as antibody B). That is, the antibody B recognizes a part or all of the amino acid sequence between the 19th amino acid residue, Ser, and the C-terminal amino acid residue Tyr in α-ANP, preferably a fragment composed of, at least, 5 amino acid residues in the amino acid sequence.

Each of antibodies A and B may be a monoclonal or polyclonal antibody.

The antibodies can be produced as follows:

The antibodies A and B can be obtained by inoculating an immunogen which is obtained by coupling a peptide derivative represented by the above formula (I) and a high molecular carrier material to a warm-blooded animal other than human beings to produce the desired antibody and collecting the antibody

thus produced.

Coupling of the peptide and the high molecular carrier material can be carried out by a conventional method [e.g. Hormone and Metabolic Research, 8, 241 (1976)]. Examples of the reagent used for the coupling include glutaraldehyde and water-soluble carbodiimide. Examples of the high polymer carrier material include bovine thyroglobulin, bovine serum albumin, bovine gammaglobulin and hemocyanine. The mixing ratio by weight of the peptide and the high polymer carrier material is suitably between 1 : 1 to 1 : 4. Good results are often obtained when the reaction pH is around neutral, particularly, about 7.3. Preferably, the reaction time is 2 to 6 hours, particularly, 3 hours. The coupled product thus produced is dialyzed against water at about 4°C according to a conventional method. If necessary, it can be frozen or lyophilized for storage.

The antibody B which recognizes the C-terminal region of α-ANP can be produced, for example, as follows:

An immunogen, for example, that obtained by coupling the peptide derivative represented by the above formula (I) and the high polymer carrier material is inoculated to several to dozens of a warm-blooded animal other than human beings to produce the antibodies. Further, a hybridoma is obtained by cell fusion and its culture supernatant or ascites fluid is collected. To select the C-terminal recognizing antibody B from these antibodies, a labeled peptide fragment is prepared by labeling a peptide fragment composed of the sequence of about 5 to 10 amino acid residues in the C-terminal region of α-ANP with a radioisotope, enzyme, fluorescent substance, luminescent substance or other labeling agent according to a known method. For example, in the case of employing enzyme immunoassay, an antibody against the immunoglobulin of the immunized animal is immobilized to a solid phase according to a standard method (the use of a 96-well microtiter plate is advantageous because quick determination using multiscan, etc. can be employed) and reacted with diluted solutions of the culture supernatant or serum of immunized animal to be tested having various degrees of dilution at a constant temperature (4 to 40°C; hereinafter, "constant temperature" means this temperature) for a predetermined period of time. The reaction product is then thoroughly washed and reacted with the above-prepared enzyme-labeled peptide fragment at a constant temperature for a predetermined period of time. After thoroughly washing, the reaction product is reacted with an enzyme substrate at a constant temperature for a predetermined period of time, followed by determination of the resulting coloring product by absorbance, fluorescence intensity or other parameters.

In the above determination, the supernatant or immunized animal serum that exhibits high absorbance or fluorescence intensity even at a high degree of dilution can be selected as the antibody B which recognizes the C-terminal region of ANP.

Further, in another method for obtaining the antibody B, the above-described solid phase coupled with the peptide fragment of C-terminal region of α-ANP is packed in a column and antibodies in a buffer solution at about neutral are adsorbed to the solid phase. After washing the column with the same buffer solution, the specifically adsorbed antibody is eluted.

For elution of the specifically adsorbed antibody, a low-pH or high-pH buffer solution or a buffer solution having a high salt concentration is used.

Examples of the low-pH buffer solution include 0.17M glycine-HCℓ buffer solution at pH 2.3 and 0.1 M sodium secondary citrate-HCℓ buffer solution at pH 1.8.

Examples of the high-pH buffer solution include aqueous ammonia at pH 11 and 0.2 M sodium borate buffer solution at pH 11.7.

Examples of the buffer solution having a high salt concentration include 6M guanidine hydrochloride solution and 7 M urea solution.

The above elution can be carried out batch-wise or by using a column.

The antibody eluate is purified, for example, by dialysis. When a low-pH buffer solution is used for elution, for example, the eluate is dialyzed against a 0.02 M phosphate-NaCℓ buffer solution (pH 8.0) containing 0.1% $NaN_3$ after neutralization with a 0.1 M sodium carbonate buffer solution (pH 10.5). When a high-pH buffer solution is used for elution, for example, the eluate is dialyzed against a 0.02 M phosphate-NaCℓ buffer solution (pH 8.0) containing 0.1% $NaN_3$ after neutralization with a 0.1M glycine-HCℓ buffer solution (pH 3.0). The antibody eluate obtained by elution with a buffer solution having a high salt concentration may be stored as it is after dialysis against the above phosphate-NaCℓ buffer solution. The resulting eluate or dialyzate can also be stored in the form of a lyophilized authentic sample preparation. Thus, the antibody B is obtained. The antibody B can be further purified according to known methods.

In order to obtain the antibody A of the present invention which recognizes the amino acid sequence between Asp and another amino acid residue in the C-terminal side in the cyclic region of α-ANP, the antibody can be advantageously selected, for example, by sandwich EIA. An antibody against the immunoglobulin of the immunized animal is immobilized onto a solid phase according to a conventional method (using a 96-well microtiter plate) and reacted with diluted solutions of the liquid containing the target antibody (e.g. culture supernatant, ascites fluid, immunized animal serum) at a constant temperature for a predetermined period of time. After washing, the reaction product is reacted with each of α-hANP and α-rANP at a constant temperature for a predetermined period of time. After thorough washing, the reaction product is reacted with a solution of the enzyme-labeled antibody B at a constant temperature for a predetermined period. The resulting coloring product is then determined by absorbance, fluorescence intensity or other parameters. In the above determination, among the culture supernatants, ascites fluids, immunized animal sera and the like that exhibit high values of absorbance or fluorescene intensity for both α-hANP and α-rANP, an antibody that does not react with the about 10 amino acid residues in the N-terminal side of α-hANP is selected as the antibody A. The

antibody A can be further purified by known methods.

It is also possible to select the antibody B by sandwich EIA after obtaining the antibody A by the above method.

Examples of the warm-blooded animal used to produce the above antibodies include mammals (e.g. rabbits, sheep, bovines, rats, mice, horses, swine) and birds (e.g. chickens, pigeons, domestic ducks, geese, quails).

The immunogen is inoculated to the warm-blooded animal at doses effective for antibody production. For example, the antibody is often produced by subcutaneously inoculating 1 mg of the immunogen emulsified with 1 mℓ of physiological saline and Freund's complete adjuvant to a rabbit at the back and hind paw pad 5 times at intervals of 4 weeks.

For collection of the antibody produced in the immunized warm-blooded animal, in the case of using a rabbit, usually, a blood sample is collected from the ear vein between 7 and 12 days following the final inoculation and then centrifuged to separate serum. Usually, the antiserum thus obtained is subjected to affinity chromatography using a carrier coupled with the antigen peptide and the adsorbed fraction is recovered to yield a purified polyclonal antibody. In addition to the polyclonal antibody, the monoclonal antibody obtained by the method of Milstein et al., Nature, 256, 495 (1975) can also be advantageously used.

In order to obtain the monoclonal antibody, rats and mice are preferred. For example, when a mouse is immunized, it is preferred to use subcutaneous, intraperitoneal or intravenous injection (subcutaneous injection is especially preferred), although any route can be used, including subcutaneous, intraperitoneal, intravenous, intramuscular, and intracutaneous injections. The interval and amount of inoculation can be also widely varied. However, usually, inoculation is is made 2 to 8 times at intervals of 2 weeks and, 1 to 5 days, preferably, 2 to 4 days after the final immunization challenge, the spleen cells are used. It is desired that the amount of inoculation is not less than 0.1µg, preferably 10 to 30µg, per mouse at each time calculated as the amount of the polypeptide.

When the spleen is removed to use the spleen cells as the lymphocyte source, it is desired that local blood sampling is conducted before removal of the spleen to confirm the increase in blood antibody titre and then fusion experiment is carried out.

The above-described cell fusion between lympocytes and a lymphoid cell line is carried out by fusing lymphocytes of an immunized mouse (preferably derived from spleen cells) with a lymphoid cell line such as myeloma of an appropriate homologous or heterologous animal (preferably homologous) having a marker such as hypoxanthine-guanine-phosphoribosyl transferase deficiency ($HGPRT^-$) or thymidine kinase deficiency ($TK^-$). For the fusion, a fusogen such as Sendai virus (HVJ), polyethylene glycol (PEG) or the like is used. It is of course possible to add fusion promoters such as dimethyl sulfoxide (DMSO) or the like. The degree of polymerization of the PEG is usually 1000 to 6000 and PEG treatment is usually carried out for 0.5 to 30 minutes in the concentration of 10 to 80%. For example, fusion can be efficiently carried out by using PEG 6000 in the concentration of 35 to 55% for 4 to 10 minutes. The fused cells (hybridoma) can be selectively proliferated using a hypoxanthine-aminopterin-thymidine medium (HAT medium) [Nature, 256, 495-497 (1975)] or the like.

The mouse serum or culture supernatant of proliferated cells can be screened for the production of the desired antibody. Antibody titre screening can be carried out as follows:

That is, radioimmunoassay (RIA), enzyme immunoassay (EIA) and other methods can be used to determine the antibody titre. These methods can be widely modified. As a preferred example of the determination method, the method based on EIA is described below. Anti-mouse immunoglobulin is immobilized to a solid phase according to a standard method (the use of a 96-well microtiter plate is advantageous because quick determination using multiscan, etc. can be employed) and reacted with the culture supernatant or mouse serum to be tested at a constant temperature for a predetermined period of time. After thoroughly washing, the reaction product is reacted with an enzyme-labeled α-ANP at a constant temperature for a predetermined period of time. After thoroughly washing, the reaction product is reacted with an enzyme substrate at a constant temperature for a predetermined period of time. The resulting coloring product is determined by absorbance, fluorescence intensity or the like.

Since α-ANP has relatively low molecular weight, it is of importance to select and combine appropriate antibodies that permit easy sandwiching. For this purpose, in the above EIA, the culture supernatant or mouse serum is firstly reacted for a predetermined period of time and then, after thoroughly washing, the reaction product is reacted with α-ANP at a constant temperature for a predetermined period of time. Further, the reaction product is reacted with an enzyme-labeled antibody (anti- α-ANP monoclonal or polyclonal antibody coupled with an enzyme such as horseradish peroxidase) at a constant temperature for a predetermined period of time. After thoroughly washing, the reaction product is reacted with an enzyme substrate at a constant temperature for a predetermined period of time. The resulting coloring product is determined by absorbance, fluorescence intensity or the like.

It is desired to clone the cells in the wells which can proliferate in a selection medium and has antibody activity against α-ANP by limiting dilution analysis or the like. The supernatant of the cloned cells is subjected to screening according to the manner as described above to increase the number of cells in the wells which show high antibody titre. Thereby, the desired monoclonal antibody producing hybridoma clone can be obtained.

The cloned hybridoma thus obtained is proliferated in a liquid medium or in the peritoneal cavity of a mammal. For example, the desired monoclonal antibody can be obtained from the culture broth after

cultivation in a liquid medium such as RPMI-1640 supplemented with 0.1 to 40% bovine serum for 2 to 10 days, preferably, 3 to 5 days. Further, a large amount of the antibody having a titre much higher than that of a cell culture supernatant can be efficiently obtained by intraperitoneally inoculating hybridoma cells to an appropriate mammal such as mouse, proliferating the cells and then collecting ascites fluid. For this purpose, for example, when mouse is used, $1 \times 10^4$ to $1 \times 10^7$, preferably, $5 \times 10^5$ to $2 \times 10^6$ hybridoma cells are intraperitoneally inoculated to Balb/C or other mice preinoculated with mineral oil or the like, and ascites fluid or the like is collected 7 to 20 days, preferably 10 to 14 days after inoculation. From the antibodies produced and accumulated in ascites fluid, the desired monoclonal antibody can be easily isolated as pure immunoglobulin, for example, by ammonia sulfate fractionation, DEAE-cellulose column chromatography or other means.

In the present invention, the above two kinds of antibodies, i.e., monoclonal and polyclonal antibodies may be immunoglobulins or their fractions [e.g., $F(ab')_2$, Fab', or Fab].

A solid phase containing one of the monoclonal and polyclonal antibodies is regarded as identical with the corresponding antibody supported by a carrier.

Examples of the carrier include gel particles such as agarose gels (e.g., Sepharose 4B and Sepharose 6B manufactured by Pharmacia Fine Chemical, Sweden), dextran gels (e.g., Sephadex G-75, Sephadex G-100 and Sephadex G-200 manufactured by Pharmacia Fine Chemical, Sweden) and polyacrylamide gels (e.g., Biogel P-30, Biogel P-60 and Biogel P-100 manufactured by Bio-Rad Laboratories, U.S.A.); cellulose particles such as Avicell (manufactured by Asahi Chemical, Japan) and ion exchange celluloses (e.g., diethylaminoethylcellulose, carboxymethylcellulose); physical adsorbents such as glass (e.g., glass balls, glass rods, aminoalkyl glass balls, aminoalkyl glass rods), silicone pieces, styrene resins (e.g., polystyrene balls, polystyrene grains); ion exchange resins such as weakly acidic cation exchange resins (e.g., Amberlite IRC-50 manufactured by Rohm & Haas, U.S.A.; Zeocarb 226 manufactured by Permutit, West Germany) and weakly basic anion exchange resins (e.g., Amberlite IR-4B manufactured by Rohm & Haas, U.S.A., Dowex 3 manufactured by Dow Chemical, U.S.A.).

Particularly, in the present invention, there can be advantageously used styrene resins such as polystyrene balls, polystyrene plates and polystyrene particles; polyvinyl chloride resins, glass, silicone pieces, polyacylamide gels or the like.

Coupling of the antibody to the carrier can be carried out by a conventional method, for example, cyanogen bromide method and glutaraldehyde method described in "Taisha (Metabolism)", 8, 696 (1971). For a simpler procedure, the antibody can be adsorbed physically to the surface of the carrier.

The labeled complex of the present invention containing the antibody other than the above monoclonal or polyclonal antibody is used in the form of a conjugate of the antibody and a labeling agent. Examples of the labeling agent include radioisotopes, enzymes, fluorescent substances and luminescent substances.

Examples of the radioisotope include $^{125}I$, $^{131}I$, $^{3}H$ and $^{14}C$. As the enzyme, it is preferred to use a stable one having high specific activity. Examples thereof include (1) carbohydrases such as glycosidases (e.g., β-galactosidase, β-glucosidase, β-glucuronidase, β-fructosidase, α-galactosidase, α-glucosidase, α-mannosidase), amylases (e.g., α-amylase, β-amylase, isoamylase, glucoamylase, takaamylase A), cellulase and lysozyme; (2) amidases (e.g., urease, asparaginase); (3) esterases such as cholinesterases (e.g., acetylcholinesterase), phosphatases (e.g., alkaline phosphatase), sulfatase and lipase; (4) nucleases (e.g., deoxy-ribonuclease, ribonuclease); (5) iron-porphyrin enzymes (e.g., catalase, peroxidase, cytochrome oxidase); (6) copper enzymes (e.g., tyrosinase, ascorbic acid oxidase); and (7) dehydrogenases (e.g., alcohol dehydrogenase, malic acid dehydrogenase, lactic acid dhydrogenase, isocitric acid dehydrogenase). Examples of the fluorescent substance include fluorescamine and fluorescence isothiocyanate. Examples of the luminescent substance include luminol, luminol derivatives, luciferin and lucignin. Among these labeliing agents, enzymes, particularly, peroxidase, is preferably used.

In the present invention, it is preferred to use the solid phase containing the monoclonal antibody and the labeled complex containing the polyclonal antibody. It is also preferred that the polyclonal antibody is used in the form of an antibody fraction (Fab' is especially preferred).

By using this case, the method for producing the labeled complex is illustrated in more detail below. However, the labeled complex can be also produced by a known method in the same manner even when using the solid phase containing the polyclonal antibody and the labeled complex containing the monoclonal antibody.

Examples of the method for coupling the polyclonal antibody and the labeling agent include conventiona methods such as chloramine T method [Nature, 194, 495 (1962)], periodic acid method [Journal of Histochemistry and Cytochemistry, 22, 1084 (1974)], maleimide method [Journal of Biochemistry, 79, 233 (1976)] and the like. When peroxidase is used as the labeling agent, particularly, it is preferred to use a binder represented by the general formula:

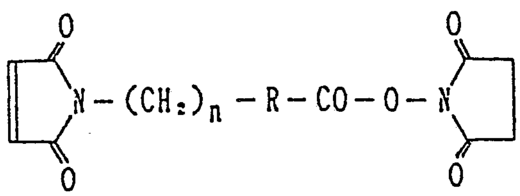

(II)

wherein n is an integer of 0 to 5; R is a chemical bond or a 6 membered cyclic hydrocarbon residue as described in Japanese Patent Laid Open Publication No. 149700/1983.

Foe example, a kit for the method of immunochemical assay of ANP of the present invention comprises:

(1) the monoclonal antibody coupled to a carrier,

(2) the labeled polyclonal antibody,

(3) 0 to 10 ng of authentic ANPs (e.g. α-hANP or α-rANP),

(4) a buffer solution for dilution of the above reagents (1) through (3) and a sample to be tested (e.g., phosphate buffer solution or glycine buffer solution at pH 6 to 9),

(5) a buffer solution for washing a carrier after incubation [any buffer solution can be used as long as it can be used to wash the carrier (e.g., phosphate buffer solution, glycine buffer solution)],

(6) reagents for determination of an enzyme in the case of using the enzyme as the labeling agent. When peroxidase is used, the reagents include reagents for peroxiodase activity determination, o-phenylenediamine and hydrogen peroxide for calorimetry and a buffer solution to dissolve an enzyme substrate (preferably, a citrate buffer solution) and a reaction terminator solution.

The composition of the buffer solution used to dilute the mentioned of the above (4) is important from the viewpoint of ANP recovery, measurement precision and the like. The present inventors have studied intensively and found that it is very effective to add 0.1 to 1% of polyethylene glycol and 0.01% to 0.1% of methylcellulose to the buffer solution.

The above kit can, for example, be used by the following method (2-step method).

About 10 to 200 μℓ of an authentic ANP preparation or a sample to be tested is diluted with the above reagent (4) and brought into contact with a predetermined amount of the reagent (1). The mixture is reacted at 0 to 40°C for 1 to 48 hours. After washing the carrier with water, about 10 to 300 μℓ of the reagent (2) is added and the mixture is further reacted at 0 to 40°C for 1 to 48 hours. After completion of the reaction, the reaction product is washed with the reagent (5) and the activity of the carrier-captured portion of the labeling agent is determined. When the labeling agent is radioisotope, a well counter or liquid scintillation counter is used to determine the labeling agent activity. When the labeling agent is an enzyme, about 10 to 1000 μℓ of a substrate is added and the mixture is reacted at 20 to 40°C for 0.2 to 24 hours. Then, the enzymatic reaction is terminated and the optical absorbance or fluorescence intensity of the reaction mixture is determined.

Alternatively, the following 1-step method can be carried out. According to this method, the reagents (1) and (2) are added at the same time to omit water washing procedure in the intermediate stage.

In the ANP assay method of the present invention, the characteristics of the antibodies used are clearly defined and the labeled complex has high sensitivity, which permits the determination with extremely high sensitivity and high reliability by exact recognition of the active site of ANPs (e.g., α-hANP, α-rANP). Further, the assay method of the present invention is very advantageous because determination of both hANP and rANP can be carried out by a single determination kit with with almost equal sensitivity and there is no need to use a different kit depending upon the objective ANP to be tested.

The following Examples and Reference Example further illustrate the present invention is in more detail but are not to be construed to limit the scope thereof.

The mouse hybridoma HA1-53-165 disclosed in the Examples has been deposited at the Institute for Fermentation, Osaka, under accession number of IFO 50169 since June 23, 1988, and Budapest Treaty, at the Fermentation Research Institute (FRI), Agency of Industrial Science and Technology, Ministry of International Trade and Industry, Japan under Budapest Treaty and accession number of FERM BP-1951 since July 12, 1988.

Example 1

Preparation of anti-α-hANP C-terminal antibody

To a solution of 12 mg of α-hANP [6-28] and 36 mg of bovine tyroglobulin (BTG) in 6 mℓ of a 0.1 M phosphate buffer solution (pH 7.0) was added dropwise 1.5 mℓ of a 1% glutaraldehyde solution with ice-cooling. After stirring at room temperature for 3 hours, the mixture was dialyzed against a physiological saline at 4°C for 20 hours. After dilution with a physiological saline to 6 mℓ, the dialyzate was distributed to 2 mℓ portions and stored in a freezer at -40°C to obtain an immunogen.

A 2-mℓ portion of this immunogen was mixed with 2 mℓ of Freund's complete adjuvant (FCA) and subcutaneously injected to 4 domestic rabbits at 1 mℓ per rabbit. In all, 5 immunization challenges were conducted at intervals of 3 weeks with the last 4 challenges using Freund's incomplete adjuvant (FIA) instead of FCA. In the next week, blood was collected to obtain rabbit.

Example 2

Preparation of labeled antibody

A 5-mℓ portion of the rabbit antiserum obtained in Example 1 was subjected to salting-out with a 45%-saturated ammonium sulfate. The resulting precipitate was dissolved in 0.02 M borate buffer solution (BBS) (pH 8.5) containing 0.15 M NaCℓ and dialyzed against BBS at 4°C for 20 hours. This dialyzate was passed through a column (1 × 0.5 cm) of Sepharose 4B (manufactured by Pharmacia, Sweden) coupled with α-hANP [6-28]. After thoroughly washing the column with BBS, elution was conducted with a 0.1 M glycine-HCℓ buffer solution (pH 2.0). The fraction of rabbit anti-α-hANP [6-28] polyclonal antibody (R3) was then collected.

The fraction thus obtained was dialyzed against a 0.1 M acetate buffer solution (pH 4.5) containing 0.1 M NaCℓ at 4°C for 20 hours. This dialyzate was digested with pepsine (0.1 mg) at 37°C for 8 hours. After stopping the reaction by addition of 1 M Tris to reach pH 8, the reaction product was applied to a column (1 × 60 cm) of Ultrogel AcA44 (manufactured by IBF, France) and the active fraction was eluted with BBS to obtain $F(ab')_2$.

After concentration, this $F(ab')_2$ was dialyzed against a 0.1 M phosphatase buffer solution (pH 6.0) at 4°C for 20 hours. This dialyzate was reduced with 0.2 mℓ of a 0.1 M phosphate buffer solution (pH 6.0) containing 0.2 M mercaptoethylamine and 5 mMEDTA at 37°C for 90 minutes. The reaction product was applied to a column (1 × 60 cm) of Sephadex G-25 Fine (manufactured by Pharmacia, Sweden) and eluted with 0.1 M phosphate buffer solution (pH 6.0) containing 5 mM EDTA to obtain an Fab' fraction. Separately, 10 mg of horseradish peroxidase (HRP) was dissolved in 1.5 mℓ of a 0.1 M phosphate buffer solution (pH 7.0). To this solution was added a solution of 3.5 mg of N-(γ-maleimidobutyloxy)succinimide (GMBS) in 100 μℓ of DMF. After stirring at 30°C for 60 minutes, this mixture was applied to a column (1.2 × 60 cm) of Sephadex G-25 fine and eluted with 0.1 M phosphate buffer solution (pH 7.0) to obtain a maleimide group introducing HRP (maleimidated HRP). The Fab' fraction and the maleimidated HRP were mixed together at a molar ratio of 1 : 1 and the mixture was reacted at 4°C for 20 hours. The reaction mixture was applied to a column (1.2 × 70 cm) of Ultrogel AcA44 and eluted with 0.1 M phosphate buffer solution (pH 7.0) to obtain an enzyme-labeled antibody (R3-HRP).

Reference Example

Preparation of α-hANP [6-28] labeled with horseradish peroxidase

1 mg of α-hANP [6-28] was dissolved in a 0.1 M phosphate buffer solution (pH 7.0). This solution was mixed with a solution of 2.4 mg of N-(γ-maleimidobutyloxy)succinimide (GMBS) in 0.1 mℓ of DMF. After stirring at 30°C for 1 hour, this mixture was applied to a column (1 × 50 cm) of Sephadex G-10 (produced by Pharmacia, Sweden) and eluted with 0.1 M phosphate buffer solution (pH 6.7) to obtain maleimidated α-hANP [6-28].

Separately, 10 mg of horseradish peroxidase (HRP) was dissolved in 1 mℓ of 0.02 M phosphate buffer solution (pH 6.8) containing 0.1 M NaCℓ. To this sollution was added a solution of 1.2 mg of N-succinimidyl-3-(2-pyridyldithio) propionate (SPDP) in 100 μℓ of ethanol, followed by stirring at room temperature for 40 minutes. To this mixture was added a solution of 5 mg of dithiothreitol (DTT) in 0.5 mℓ of a 0.1 M acetate buffer solution (pH 4.5), followed by stirring at room temperature for 20 minutes. This mixture was applied to a column (1 × 50 cm) of Sephadex G-25 fine (manufactured by Pharmacia, Sweden) and elulted with 0.1 M phosphate buffer solution (pH 6.8) containing 2 mM EDTA to obtain thiolated HRP.

The maleimidated α-hANP [6-28] and the thiolated HRP were mixed together at a molar ratio of 5 : 1. After maintaining at 4°C for 24 hours, this mixture was applied to a column (1 × 60 cm) of Ultrogel AcA44 (manufactured by IBF, France) and eluted with 0.1 M phosphate buffer solution (pH 6.8) to obtain α-hANP [6-28] labeled with horseradish peroxidase.

Example 3

Preparation of anti-α-hANP monoclonal antibody for recognizing cyclic region

(Immunization)

0.5 mg of α-hANP and 5 mg of BTG were dissolved in 0.5 mℓ of distilled water. To this solution was added dropwise a solution of 3 mg of 1-ethyl-3-(3-dimethylaminopropyl)carbodiimide hydrochloride (ECDI) in 0.1 mℓ of distilled water. After stirring at room temperature for 2 hours, the mixture was dialyzed against a physiological saline at 4°C for 20 hours. This dialyzate was diluted with physiological saline to 10 mℓ and this solution was distributed to 1-mℓ portions and stored in a freezer at -40°C.

A 1-mℓ portion of this immunogen was mixed with 1.5 mℓ of FCA and subcutaneously injected to 8 mice (Balb/c line, 7-week old) at 200 μℓ per animal. After 3 weeks, the second immunization was conducted using FIA. After one week, local blood sampling was conducted and the antibody titre of each serum sample was then determined. After additional 2 weeks, the mice found to have serum with high antibody titre were further dosed with 200 μℓ of the immunogen as the final immunization challenge via tail vein. The antibody titre was determined as follows:

To immunoplates coupled with anti-mouse immunoglobulin were injected 10-fold serial dilutions (the initial

degree of dilution was 100 times) of each serum diluted with 0.02 M phosphate buffer solution (pH 6.8) containing 10% calf serum. Further, α-hANP [6-28] labeled with horseradish peroxidase was added. Then, the mixture was incubated at room temperature for 2 hours. After washing the immunoplates with a 0.02 M phosphate buffer solution (pH 7.0) containing 0.15 M NaCℓ, a 0.1 M citrate buffer solution (pH 4.9) containing 0.2% o-phenylenediamine (OPD), 0.02% $H_2O_2$ and 0.002% merthiolate was added and a coloring reaction is carried out at room temperature for 10 minutes. Optical absorbance at 492 nm was then determined.

Optical absorbance decreases as a degree of dilution increases. It can be said that, when a degree of dilution the optical absorbance of which is corresponding to one half of the maximum value becomes higher, the antibody has a higher titre.

(Preparation of monoclonal antibody producer cell line)

Cell fusion was conducted by the method of Milstein et al. as follows:

Spleens were excised from immunized mice 4 days after the final immunization challenge. After thoroughly washing with MEM medium, 1 × $10^8$ spleen cells of the excised spleen and 2 × $10^7$ mouse myeloma cells (P3/X63-Ag8, UI) were mixed together and centrifuged at 700 r.p.m. for 15 minutes to obtain pellets. To these pellets was added 0.3 mℓ of a 45% (w/v) solution of polyethylene glycol 6000 in RPMI-1640. After dilution by gradual addition of 15 mℓ of RPMI-1640, the mixture was centrifuged at 700 r.p.m. for 15 minutes and, then, the cells were dispersed in 100 mℓ of an RPMI-1640 medium containing 20% fetal bovine serum. A 2 mℓ portions of this cell dispersion were distributed to wells of a 24-well culture plate (manufactured by Flow Co., U.S.A.). At 2nd, 5th and 8th days following the initiation of cultivation, one half of the culture supernatant was replaced with HAT medium (RPMI-1640 medium containing 1 × $10^{4}$ M hypoxanthine, 4 × $10^{7}$ M aminopterin, 1.6 × 10 $^{5}$ M thymidine and 20% fetal bovine serum). After 14 days, the screening system as described hereinafter was used to select IgG producing lines which permit sandwich EIA of α-hANP. The selected lines were then cloned by repeating the limiting dilution procedure using an RPMI-1640 medium containing 20% fetal bovine serum and Balb/c mouse thymocytes as the feeder to eventually obtain the mouse hybridoma HA1-53-165 line (IFO 50169, FERM BP-1951).

Screening system for monoclonal antibody

Anti-mouse IgG coupled plate

↓ Culture supernatant, 100 μℓ/well

↓ Room temperature, 2 hours

↓ Washing with PBS

↓ α-hANP standard solution (10 ng/mℓ), 100 μℓ/well

↓ Room temperature, 2 hours

↓ Washing with PBS

↓ α-hANP antibody (Fab′) labeled with horseradish peroxidase (R3-HRP)

↓ Room temperature, 2 hours

↓ Coloring reaction

Determination of absorbance at 492 nm

(Purification of monoclonal antibody HA53)

The hybridoma HA1-53-165 line was intraperitoneally injected to Balb/c mice. IgG was purified from each ascites fluid sample by a conventional method. That is, 10 mℓ of ascites fluid was subjected to salting-out with 45%-saturated ammonium sulfate. The resulting precipitate was dissolved in BBS and dialyzed against BBS at 4°C for 20 hours. This dialyzate was applied to a column of DE-50 (1 × 60 cm, manufactured by Whatman, U.K.) and eluted by the density gradient elution method with a linear gradient of NaCℓ density of from 0.1 M to 0.35 M in a 0.1 M phosphate buffer solution (pH 8.0) to obtain 150 mg of monoclonal antibody HA 53.

Example 4

Deduction of recognition site of polyclonal antibody (R3)

To each well of an immunoplate coupled with anti-rabbit IgG were added the rabbit anti-α-hANP [6-28] polyclonal antibody (R3) obtained in Example 2, the α-hANP [6-28] labeled with horseradish peroxidase obtained in the Reference Example and a competing agent listed in Table 1, followed by incubation at 37°C for 2 hours. After washing immunoplate with a 0.02 M phosphate buffer solution (pH 7.0) containing 0.15 M NaCℓ, a 0.1 M citrate buffer solution (pH 4.9) containing 0.2% o-phenylenediamine (OPD), 0.02% $H_2O_2$ and 0.002% merthiolate was added as the coloring substrate (the same buffer solution was used as the coloring substrate in the procedures described below as well), followed by coloring reaction at room temperature for 30 minutes. Optical absorbance at 492 nm was then determined. The ratio of decrease in absorbance in each well relative to the absorbance obtained in the absence of competing agent was calculated and taken as the ratio of α-hANP-antibody reaction inhibition by each competing agent. The results are shown in Fig. 1. The decrease in the inhibitory ratio is due to a lack of antibody recognition site in the competing agent. As seen Fig. 1, it is considered that the polyclonal antibody (R3) obtained in Example 2 recognizes the C-terminal side of α-hANP.

Table 1

| Competing agent number | |
|---|---|
| 1 | [des $Gly^9$] α-hANP [7-28] |
| 2 | [des 9-10] α-hANP [7-28] |
| 3 | [des 11-13] α-hANP [7-28] |
| 4 | [des $Asp^{13}$] α-hANP [7-28] |
| 5 | [des 17-20] α-hANP [7-28] |
| 6 | [des 21-22] α-hANP [7-28] |
| 7 | α-rANP |

Example 5

Deduction of recognition site of monoclonal antibody (HA53)

To each well of an immunoplate coupled with mouse anti-α-hANP monoclonal antibody were added the α-hANP [6-28] labeled with horseradish peroxidase, obtained in the Reference Example, and a competing agent listed in Table 1 above, followed by incubation at 37°C for 2 hours. After washing the immunoplate with a 0.02 M phosphate buffer solution (pH 7.0) containing 0.15 M NaCℓ, the above-described coloring substrate was added, followed by coloring reaction at room temperature for 30 minutes. Optical absorbance at 492 nm was then determined. The inhibitory ratio was calculated from the absorbance according to the same manner as described in Example 4. The results are shown in Fig. 2. As seen from Fig. 2, it is considered that the monoclonal antibody (HA53) obtained in Example 3 recognizes the cyclic region of α-hANP.

Further, to each well of an immunoplate coupled with the mouse anti- α-hANP monoclonal antibody were added the α-hANP [6-28] labeled with horseradish perocidase, obtained in the Reference Example, and one of α-hANP [13-28], α-hANP [18-28] and α-hANP [1-28] which was reduced and then carboxymethylated (abbreviated as CM-α-hANP [1-28]), followed by incubation at 37°C for 2 hours. After washing the immunoplate with a 0.02 M phosphate buffer solution (pH 7.0) containing 0.15 M NaCℓ, the coloring substrate was added, followed by coloring reaction at room temperature for 30 minutes. Optical absorbance at 492 nm was then determined. Cross reactivity to α-hANP [13-28], α-hANP [18-28] or CM-α-hANP [1-28] was calculated from the absorbance. The results are shown in Figs. 3 and 4. According to the ressults, it is considered that, for the monoclonal antibody (HA53) obtained in Example 3, the disulfide bond between Cys at 7-position and Cys at 23-position of α-hANP is not an important recognition site.

Example 6

Cross reactivity of α-hANP and α-rANP

To each well of an immunoplate coupled with the monoclonal antibody HA53 obtained in Example 3 was added 100 μℓ of a 0 to 500 pg/mℓ α-hANP or α-rANP. After standing at 20°C for 2 hours, the immunoplate was washed with a 0.02 M phosphate buffer solution (PBS) containing 0.15 M NaCℓ. The enzyme-labeled antibody R3-HRP obtained in Example 2 was then added at 100 μℓ per well. After standing at 20°C for 2 hours, the immunoplate was washed with PBS. The same coloring substrate as in Example 4 was added, followed by coloring reaction at 20°C for 30 minutes. After stopping the reaction by addition of 1 N $H_2SO_4$ at 100 μℓ per well, absorbance at 492 nm was determined. The results are shown in Fig. 5. As seen from Fig. 5, it is clear that the sandwich EIA system using a combination of the monoclonal antibody HA53 and the polyclonal antibody R3 exhibits similar reactivities to α-hANP and α-rANP.

Example 7

Determination of ANPs

(Buffer solutions)

The buffer solutions used to determined α-hANP or α-rANP had the following compositions:

| | |
|---|---|
| Buffer solution A: | 0.1 M phosphate buffer solution (pH 7.0), 0.1 M NaCℓ, 1 mM $MgCℓ_2$, 0.1% bovine serum albumin (BSA), 200 U/mℓ aprotinin, 0.5% polyethylene glycol 6000, 0.05% methylcellulose 4000 cP, 0.005% merthiolate |
| Buffer solution B: | 0.1 M phosphate buffer solution (pH 7.0), 0.1% BSA, 0.1% Tween 20 |
| Washing solution: | PBS |
| Coloring substrate: | 0.1 M citrate buffer solution (pH 4.9) containing 0.2% o-phenylenediamine (OPD), 0.02% $H_2O_2$ and 0.002% merthiolate |

(Preparation of standard serum)

To 80 mℓ of human serum (subjected to heat treatment at 56°C for 30 minutes) was added 10 g of granular activated charocoal (Z-200, produced by Takeda Chemical Industries, Japan) boiled and washed with distilled water. This mixture was allowed to stand at 4°C for 1 hour. After the resulting supernatant was passed through a filter of 0.22 μm pore size, 0.005 % merthiolate and 200 U/mℓ aprotinin were added to obtain a standard human serum (hereinafter referred to as HS).

(Preparation of HA53-coupled plate)

$F(ab')_2$ of HA 53 was diluted to 20 μg/mℓ with a 0.1 M carbonate buffer solution (pH 9.5) and transferred to an immunoplate (produced by Nunc) at 100 μℓ per well. After standing at 4°C for 24 hours, the immunoplate was washed with PBS. A 0.1 M phosphate buffer solution (pH 7.0) containing 1% BSA was then added to the immunoplate at 300 μℓ per well. After standing at 4°C for more than 24 hours, the immunoplate was used as the antibody-coupled plate.

(Determination)

All determinations of ANPs were carried out in duplicate. The standard solution was prepared by adding 120 μℓ of HS to 360 μℓ of the buffer solution A containing a predetermined amount of α-hANP. The sample was prepared by adding 120 μℓ of human blood plasma to 360 μℓ of the buffer solution A. The standard solution and the sample were added to the immunoplate at 200 μℓ each per well, followed by incubation at 4°C for 24 hours. After washing immunoplate 4 times with PBS, a 100-fold dilution of the enzyme-labeled antibody (R3-HRP) in the buffer solution B was added at 100 μℓ per well, followed by incubation at 4°C for 20 hours. After immunoplate washing 6 times with PBS, the coloring substrate was added at 100 μℓ per well, followed by coloring reaction at room temperature for 30 minutes. After stopping the reaction by addition of 1 N $H_2SO_4$ at 100 μℓ per well, absorbance at 492 nm was determined.

Example 8

Determination of ANPs by competitive EIA

To each well of a plate coupled with the antibody (HA53) were added 50 μℓ of the subject solution and 50 μℓ of a solution of α-hANP [6-28] labeled with horseradish peroxidase obtained in the Reference Example. The plate was left at 4° C for 20 hours. After washing the plate 6 times with PBS, the coloring substrate was added at 100 μℓ per well, followed by coloring reaction at room temperature for 30 minutes. After stopping the reaction by addition of 1 N $H_2SO_4$ at 100 μℓ per well, absorbance at 492 nm was determined.

Example 9

Determination of ANPs by sandwich EIA (one-step method)

A 100-fold dilution of the enzyme-labeled antibody R3-HRP with the buffer solution A was added to an antibody-coupled plate at 150 μℓ per well. The standard human serum (HS) prepared in Example 7 or a human blood plasma sample was added at 50 μℓ per well. The plate was left at 4° C for 20 hours. After plate washing 6 times with PBS, the coloring substrate was added at 100 μℓ per well, followed by coloring reaction at room temperature for 30 minutes. After stopping the reaction by addition of 1 N $H_2SO_4$ at 100 μℓ per well, absorbance at 492 nm was determined.

The method of immunochemical assay using antibodies of the present invention permits determination of ANPs (e.g. α-hANP, α-rANP) with high sensitivity and high reliability.

Further, since the present method permits determination of both human ANPs (e.g. α-hANP) and rat ANPs (e.g. α-rANP) with nearly equal sensitivities by means of a single kit, there is no need to change kits according to the type of assay subjects.

Furthermore, since the recognition site of the antibodies of the present invention does not contain -S-S-bond in the cyclic region, the determination can be carried out regardless of the presence of such a bond and, therefore, sandwich technique is suitable employed.

## Claims

1. An antibody which recognizes the amino acid sequence between Asp and another amino acid residue in the C-terminal side in the cyclic region of α-ANP.

2. An antibody obtained by using as the immunogen a coupling product of a peptide derivative represented by the general formula (I):

R-Cys-Phe-Gly-Gly-Arg-$R^1$-Asp-Arg-Ile-Gly-Ala-Gln-

Ser-Gly-Leu-Gly-Cys-Asn-Ser-Phe-Arg-Tyr-OH

(I)

wherein R is hydrogen, Ser, Ser-Ser, Arg-Ser-Ser, Arg-Arg-Ser-Ser, Leu-Arg-Arg-Ser-Ser, Ser-Leu-Arg-Arg-Ser-Ser or hydrocarbon acyl having 2 to 18 carbon atoms which may be substituted with an amino group at the α-position; $R^1$ represents Met or Ile, and a high molecular substance for a carrier.

3. An antibody according to claim 2, which recognizes an amino acid sequence of the formula:
$NH_2$-Ser-Gly-Leu-Gly-Cys-Asn-Ser-Phe-Arg-Tyr-OH

4. A method of immunochemical assay of ANPs, which comprises using the antibody as claimed in claim 1 and another antibody whose antigen determinant site differs from that of the former.

5. A method of immunochemical assay according to claim 4, wherein the method is carried out by sandwich technique.

6. A method of immunochemical assay according to claim 4, wherein said another antibody recognizes the sequence of 10 amino acid residues in the C-terminal region of α-ANP.

7. A method of immunochemical assay according to claime 4, wherein the antibody as claimed in claim 2.

8. A method of immunochemical assay according to claim 5, wherein a neutral buffer solution comprising polyethylene glycol and methylcellulose is used as the diluent for antibodies and antigens.

Fig. 1

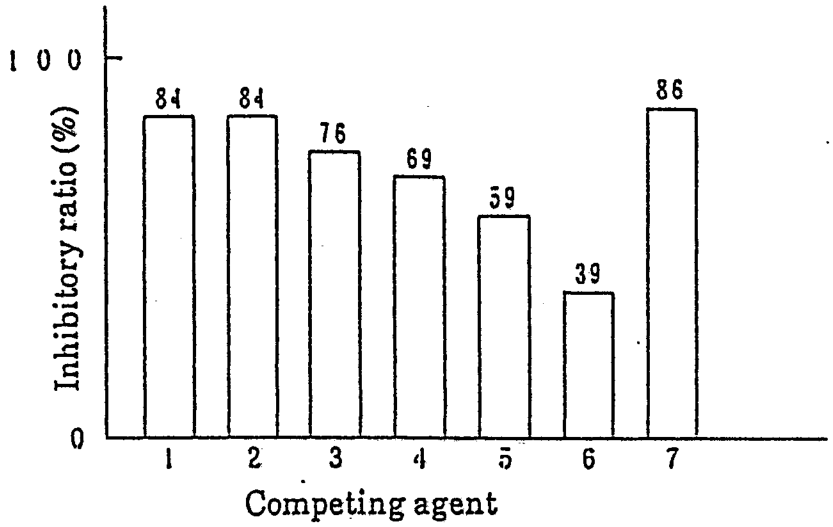
100
0
84
84
76
69
59
39
86
Inhibitory ratio (%)
1
2
3
4
5
6
7
Competing agent

Fig. 2

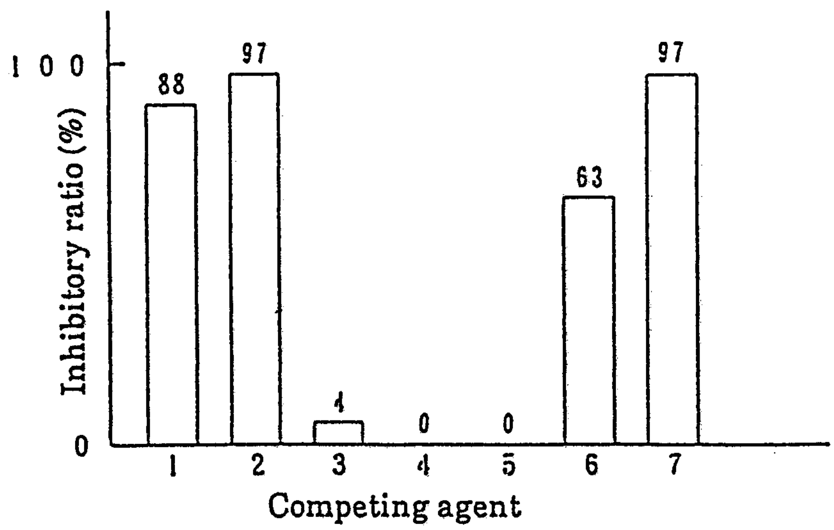
100
0
88
97
4
0
0
63
97
Inhibitory ratio (%)
1
2
3
4
5
6
7
Competing agent

Fig. 3

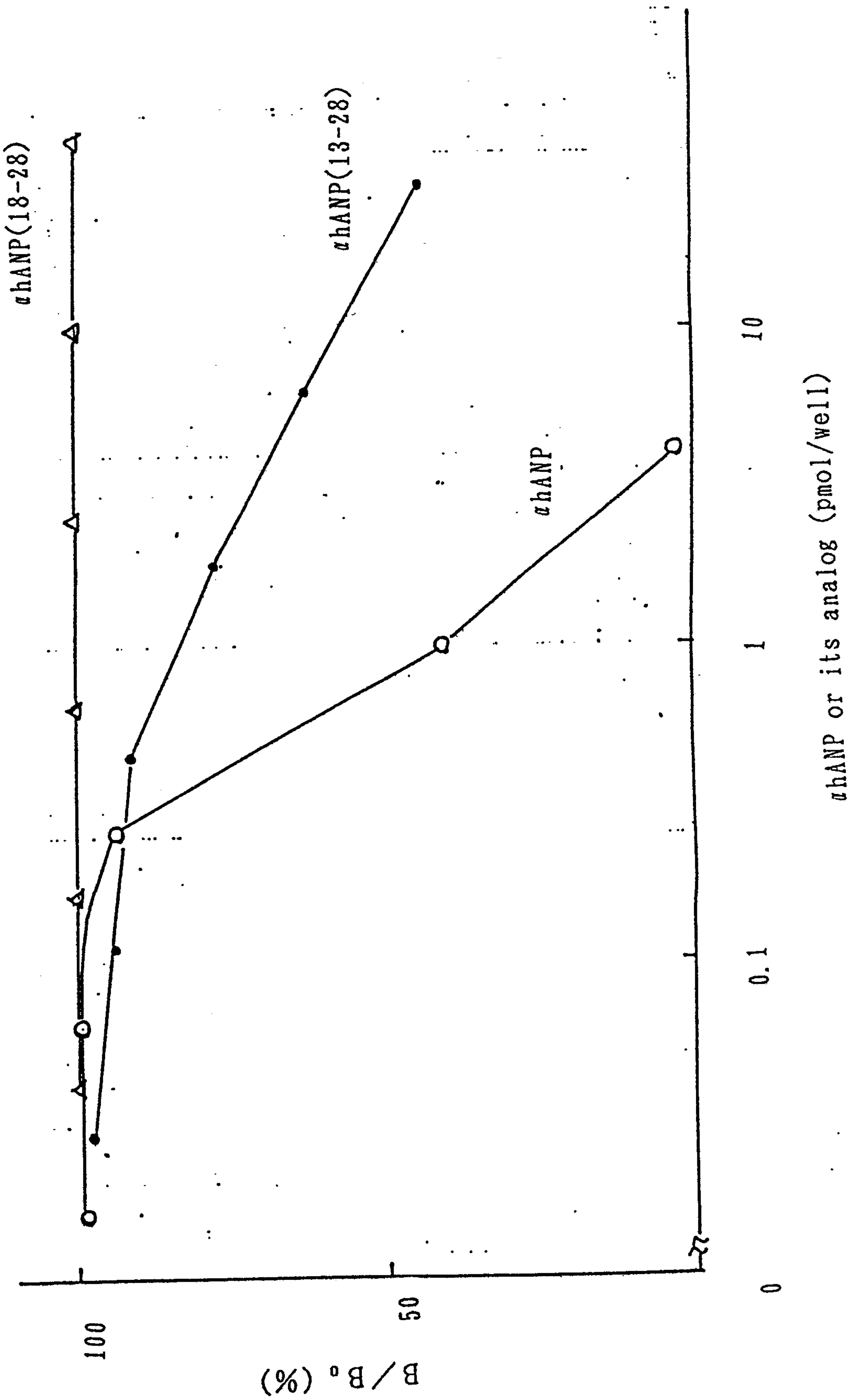
αhANP(18-28)
αhANP(13-28)
αhANP
100
50
B/B₀ (%)
0
0.1
1
10
αhANP or its analog (pmol/well)

Fig. 4

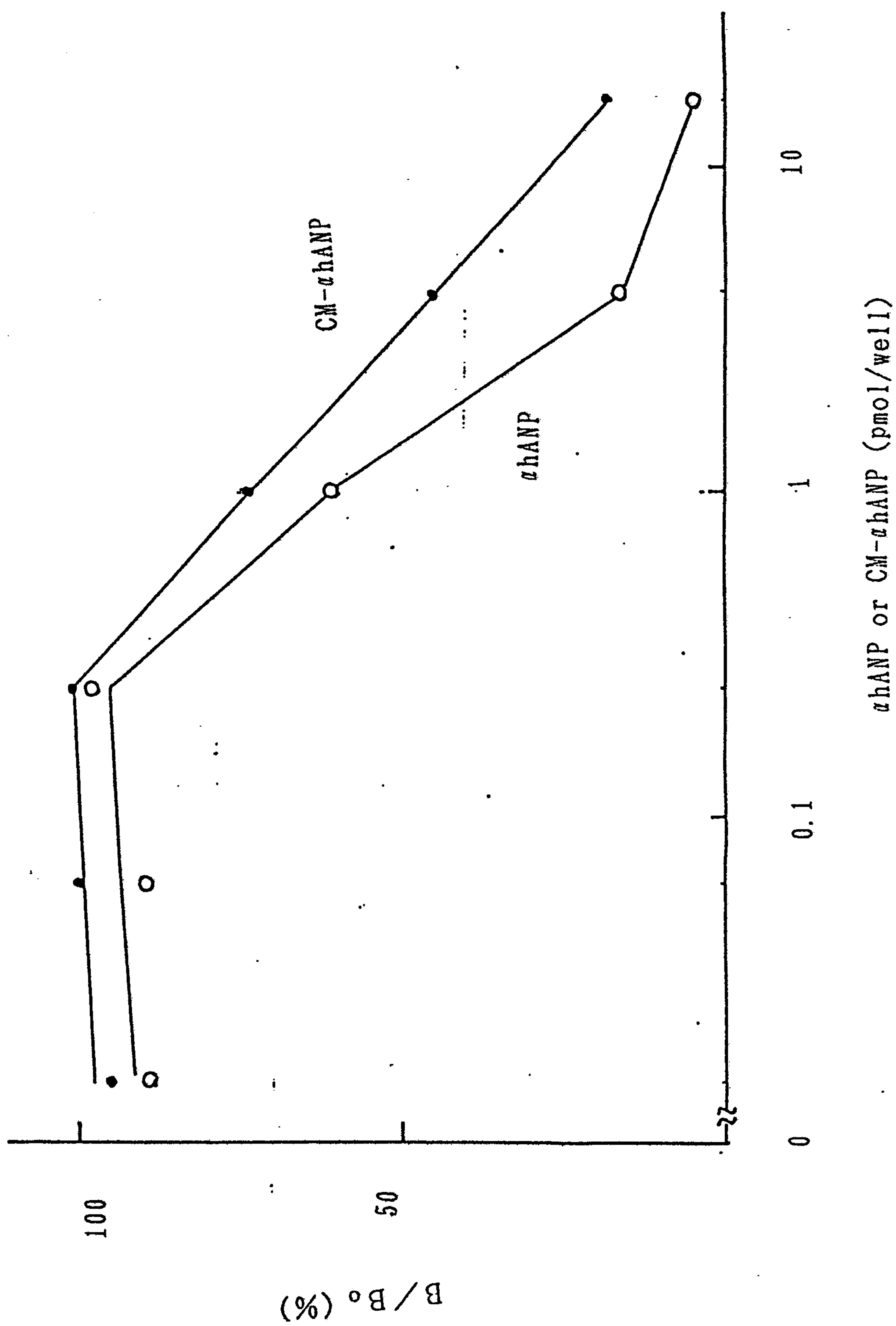

Fig. 5

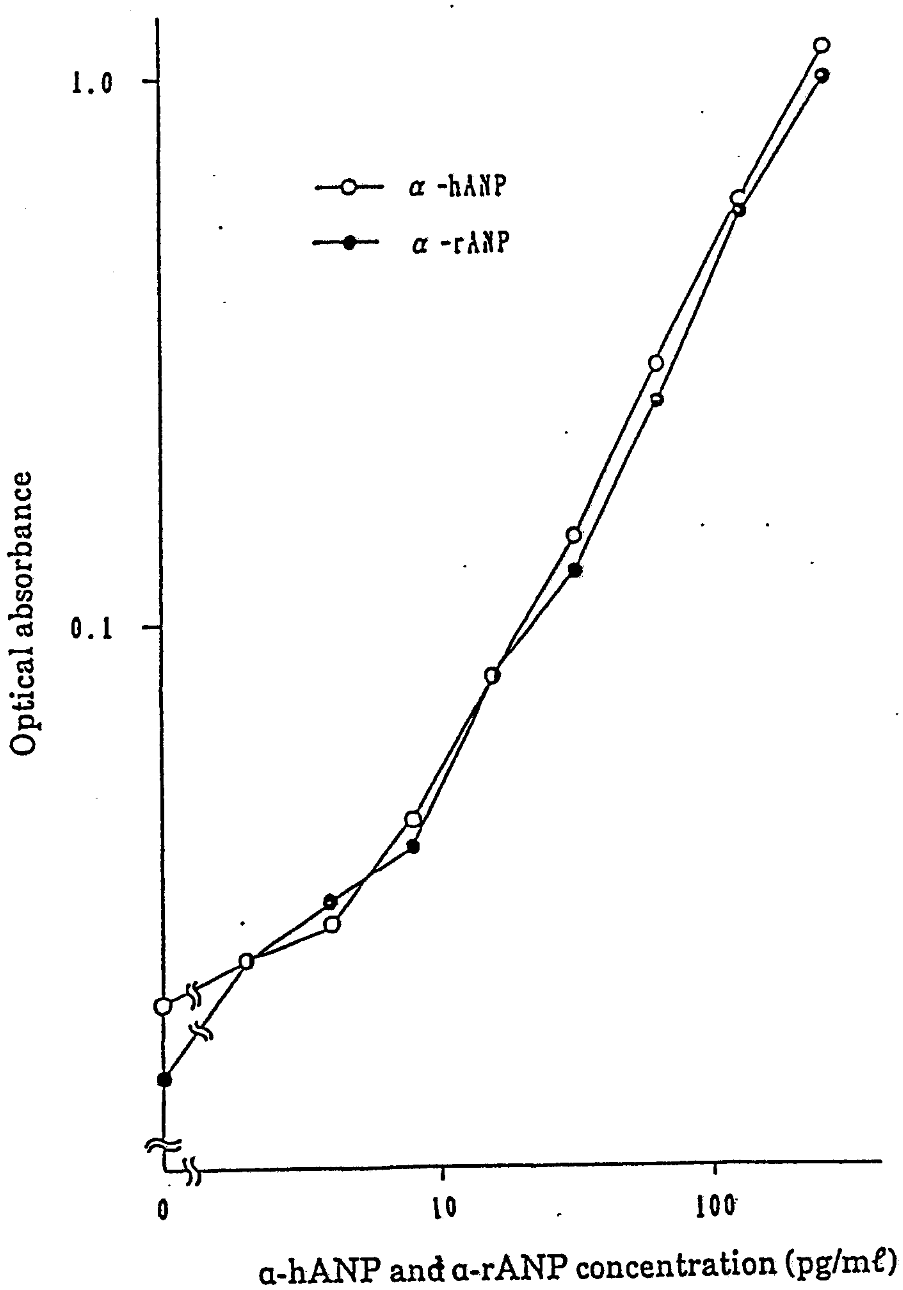
1.0
0.1
Optical absorbance
α -hANP
α -rANP
0
10
100
α-hANP and α-rANP concentration (pg/mℓ)